# DEMANDE DE BREVET EUROPEEN

(11) **EP 3 973 852 A1**
(43) Date de publication de la demande: **30.03.2022**
(21) Numéro de dépôt: 20198888.8
(22) Date de dépôt: 29.09.2020
(51) Int. Cl.: A61B 5/00

(54) **SYSTÈME CENTRALISÉ DE DÉTECTION DE LA DÉGLUTITION D'UNE PLURALITÉ D'UTILISATEURS ET PROCÉDÉ ASSOCIÉ**

(71) Demandeur: Swallis Médical, 67000 Strasbourg (FR)
(72) Inventeur: PERRIN, Nicolas, 67120 Kolbsheim (FR); NICOLINI, Linda, 34000 Toulouse (FR); NEVEU, Fabrice, 34000 Montpellier (FR)
(74) Mandataire: Cabinet Camus Lebkiri

(57) **Abrégé**

Un aspect de l'invention concerne un système centralisé d'analyse de la déglutition d'une pluralité d'utilisateurs comprenant :
- Une pluralité de dispositifs de détection de la déglutition, chaque dispositif de détection de la déglutition comprenant au moins un capteur de détection de déglutition configuré pour mesurer un signal de déglutition d'un utilisateur,
- Au moins un dispositif central de gestion connecté aux dispositifs de détection de la pluralité de dispositifs de détection et comprenant:
- un module de réception configuré pour recevoir les signaux de déglutition,
- au moins un processeur configuré pour réaliser une analyse de chacun des signaux de déglutition reçus,
- un module de stockage configuré pour stocker chacun des signaux de déglutition reçus et le résultat de l"analyse de chacun des signaux de déglutition reçus,
- un module d'affichage configuré pour afficher au moins un signal stocké et/ou le résultat de l'analyse du au moins un signal stocké.

## Description

### DOMAINE TECHNIQUE DE L'INVENTION

Le domaine technique de l'invention est celui de la détection de la déglutition.

La présente invention concerne un système de gestion d'une pluralité de dispositifs de détection de la déglutition et en particulier un système comprenant un module central de collecte et le traitement de données de déglutition de plusieurs dispositifs de détection de la déglutition.

### ARRIERE-PLAN TECHNOLOGIQUE DE L'INVENTION

Les patients atteints de troubles de la déglutition, aussi appelés « dysphagie », présentent des difficultés à avaler les aliments, par exemple le manque de coordination dans le cheminement des aliments entre la bouche et l'estomac passant par le pharynx et l'œsophage, et des risques de fausses-routes. Concernant les fausses-routes, on parle de pénétration si l'aliment entre dans le larynx mais reste au-dessus de la glotte, des cordes vocales, et d'aspiration s'il passe les cordes vocales. L'aspiration entraîne une des réponses corporelles, normalement une toux, mais s'il existe un trouble de sensibilité, l'aspiration peut être sans toux et donc silencieuse.

La dysphagie peut par exemple apparaître chez des patients après un accident vasculaire cérébral (« AVC »), après un traumatisme crânio-cérébral (« TCC »), après une Sclérose Latérale Amiotrophique ou chez les patients atteints de la maladie d'Alzheimer ou de maladie neurodégénératives.

Afin de détecter le niveau de dysphagie d'un patient, par exemple pour évaluer le risque de fausse route, le type, la cause et/ou le niveau de gravité de la dysphagie, de nombreuses méthodes d'examen ont été développées.

L'étude de l'accélérométrie de la déglutition a notamment été développée face à un besoin de méthodes non-invasives d'évaluation de la dysphagie, particulièrement grâce à la miniaturisation et à l'amélioration de la précision des accéléromètres électroniques. Des dispositifs colliers comprenant un accéléromètre ont été développés pour l'acquisition de signaux d'accélérométrie de déglutition au niveau du larynx. En effet, une déglutition peut être divisée en trois phases :
- la phase orale de la déglutition est volontaire et comprend le mouvement postérieur de la langue et de l'os hyoïde,
- la phase laryngo-pharyngée est automatique et réflexe et comprend le mouvement laryngé, l'élévation de l'os hyoïde, la fermeture de l'épiglotte et le passage du bolus vers l'orifice œsophagien, et
- la phase oesophagienne est réflexe et comprend la contraction péristaltique, le repositionnement de l'os hyoïde et du larynx et la réouverture de l'épiglotte.

La captation de ces signaux permet leur traitement par ordinateur et leur caractérisation par rapport à ces trois phases de la déglutition, et des techniques de classification automatique ont ainsi été appliquées à ces signaux dans l'état de la technique pour la détection de dysphagies, d'aspirations, de fausses-routes silencieuses et/ou pour l'évaluation du niveau de dysphagie.

De tels dispositifs colliers peuvent en outre comprendre un capteur de son laryngé, par exemple un microphone, pour la mesure du son de déglutition, par exemple afin de filtrer et d'analyser les sons de respiration, de toux et les sons liés à la voix du patient. Le microphone peut aussi permettre la détection de la déglutition, par exemple en détectant le son d'ascension laryngé correspondant à l'ascension du larynx quand le bolus est localisé dans l'oropharynx et/ou l'hypopharynx, le son d'ouverture du sphincter supérieur correspondant au transit du bolus à travers le sphincter supérieur, et le son de relâchement laryngé correspondant à la descente et à l'ouverture du larynx quand le bolus a atteint l'œsophage, comme décrit par Sylvain Morinière et al. dans « Origin of the Sound Components During Pharyngeal Swallowing in Normal Subjects », Dysphagia, 2008.

Ces dispositifs colliers peuvent être utilisés pour la détection et l'analyse de déglutitions chez un patient atteint de dysphagie, ou pour l'analyse des exercices de rééducation à la déglutition. Dans la détection et l'analyse de déglutitions, un utilisateur porte un dispositif collier pour lequel les signaux sont analysés généralement par un dispositif connecté au collier tel qu'un smartphone ou un autre dispositif électronique spécialisé. Ces dispositifs électroniques sont personnels et stockent des données médicales. Ils permettent individuellement de ne gérer qu'un seul dispositif collier de déglutition à cause de la nature sensible (médicale) des données et du fait qu'un modèle adapté à la détection apprend à partir des données précédentes de déglutition liées à l'utilisateur.

Dans les établissements médicaux par exemple, cela pose un problème car le personnel soignant doit vérifier un par un tous les dispositifs individuels des patients et peut manquer une alerte ou un avertissement quant à une fausse-route ou une aspiration silencieuse ou non.

Il existe donc un besoin de pouvoir étudier la déglutition de plusieurs patients simultanément.

### RESUME DE L'INVENTION

L'invention offre une solution aux problèmes évoqués précédemment, en permettant une étude non invasive simultanée de la déglutition de plusieurs patients.

Un aspect de l'invention concerne un système centralisé d'analyse de la déglutition d'une pluralité d'utilisateurs, le système étant caractérisé en ce qu'il comprend :
- Une pluralité de dispositifs de détection de la déglutition, chaque dispositif de détection de la déglutition comprenant au moins un capteur de détection de déglutition configuré pour mesurer un signal de déglutition d'un utilisateur de la pluralité d'utilisateurs,
- Au moins un dispositif central de gestion connecté aux dispositifs de détection de la pluralité de dispositifs de détection et comprenant au moins
   - un module de réception configuré pour recevoir les signaux de déglutition de la pluralité de dispositifs de détection de la déglutition,
   - au moins un processeur configuré pour réaliser une analyse de chacun des signaux de déglutition reçus,
   - un module de stockage configuré pour stocker chacun des signaux de déglutition reçus et le résultat de l'analyse de chacun des signaux de déglutition reçus réalisée par le processeur,
   - un module d'affichage configuré pour afficher au moins un signal stocké et/ou le résultat de l'analyse du au moins un signal stocké.

Grâce à l'invention, il est possible d'étudier la déglutition de plusieurs patients simultanément et à un même endroit. Le système selon l'invention comprend un dispositif central de gestion qui permet de gérer plusieurs dispositifs de détection de la déglutition « satellites ». On entend par « satellites » les dispositifs de détection de la déglutition connectés au dispositif central de gestion. Ainsi, lorsque plusieurs utilisateurs portent chacun un dispositif de détection de la déglutition, il est possible d'avoir accès, de manière centralisée, aux signaux de déglutition provenant des différents dispositifs de détection de la déglutition et/ou aux résultats de l'analyse de ces signaux.

L'analyse de ces signaux est avantageusement centralisée, permettant d'avoir des dispositifs de détection de la déglutition « satellites » avec moins de ressources, notamment de calcul, et donc permettant d'avoir des dispositifs de détection de la déglutition moins coûteux. Cela permet aussi de pouvoir réaliser des mises à jour d'applications logicielles d'analyse des signaux plus simplement, en n'ayant qu'une entité à mettre à jour plutôt que chaque dispositif de détection de la déglutition un par un. Enfin, l'analyse des signaux peut être réalisée en utilisant un modèle unique apprenant grâce aux données de l'ensemble des dispositifs de détection de la déglutition « satellites », ou une pluralité de modèles, chaque modèle étant dédié à un ou plusieurs utilisateurs.

Ainsi, le dispositif central de gestion permet d'afficher, analyser et stocker les signaux et l'analyse de ces signaux provenant de plusieurs dispositifs de détection de la déglutition. Il permet aussi de comparer les signaux d'utilisateurs différents, ainsi que les résultats de l'analyse de ces signaux et donc de trouver des relations entre les déglutitions analysées comme « anormales » et des événements liés à la déglutition, par exemple un même type d'aliment que plusieurs utilisateurs auraient ingurgité.

Outre les caractéristiques qui viennent d'être évoquées dans le paragraphe précédent, le système centralisé selon un aspect de l'invention peut présenter une ou plusieurs caractéristiques complémentaires parmi les suivantes, considérées individuellement ou selon toutes les combinaisons techniquement possibles :
- le dispositif central de gestion comprend en outre un module d'alarme configuré pour émettre une alarme lorsque le résultat de l'analyse d'un des signaux de déglutition reçus vérifie une règle prédéfinie.
- le module d'alarme est configuré pour vérifier la règle prédéfinie, la règle prédéfinie étant vérifiée lorsque le signal de déglutition reçu est classifié dans au moins une classe prédéfinie.
- le module de stockage est configuré pour stocker chacun des signaux de déglutition reçus et le résultat de l'analyse de chacun des signaux de déglutition reçus en association avec un identifiant utilisateur.
- le capteur de détection de déglutition est un microphone pour détecter un son de déglutition et/ou un accéléromètre pour détecter un mouvement de déglutition.
- les dispositifs de détection de la déglutition comprennent chacun en outre au moins un capteur parmi les capteurs de fréquence cardiaque, de température du corps, de sudation, de son de respiration, de fréquence respiratoire, d'activité musculaire.

Un autre aspect de l'invention concerne un procédé d'analyse de la déglutition d'une pluralité d'utilisateurs, chaque utilisateur utilisant un dispositif de détection de la déglutition, le procédé comprenant les étapes de :
- Mesure d'au moins un signal de déglutition par chaque dispositif de détection de déglutition ;
- Envoi des signaux de déglutition par chaque dispositif de détection de déglutition à un module de réception d'un dispositif central de gestion ;
- Analyse de chaque signal de déglutition reçu par un processeur du dispositif central de gestion.

Outre les caractéristiques qui viennent d'être évoquées dans le paragraphe précédent, le procédé selon un autre aspect de l'invention peut présenter une ou plusieurs caractéristiques complémentaires parmi les suivantes, considérées individuellement ou selon toutes les combinaisons techniquement possibles :
- l'étape d'analyse de chaque signal de déglutition reçu comprend la classification de chaque signal de déglutition reçu.
- le procédé d'analyse de la déglutition d'une pluralité d'utilisateurs comprend en outre une étape d'émission d'une alarme, par un module d'alarme du dispositif central de gestion, lorsqu'au moins un signal de déglutition parmi les signaux de déglutition reçus est classifié dans au moins une classe prédéfinie.

L'invention trouve un intérêt particulier dans les centres médicaux accueillant plusieurs patients atteints de troubles de la déglutition, ainsi que dans le contexte d'accès à distance à des données de déglutition par un même praticien pour plusieurs patients.

L'invention et ses différentes applications seront mieux comprises à la lecture de la description qui suit et à l'examen des figures qui l'accompagnent.

### BREVE DESCRIPTION DES FIGURES

Les figures sont présentées à titre indicatif et nullement limitatif de l'invention.
- La figure 1 montre une représentation schématique d'un système centralisé d'analyse de la déglutition d'une pluralité d'utilisateurs selon l'invention.
- La figure 2 montre une représentation schématique d'un procédé d'analyse de la déglutition d'une pluralité d'utilisateurs selon l'invention.

### DESCRIPTION DETAILLEE

Les figures sont présentées à titre indicatif et nullement limitatif de l'invention.

Sauf précision contraire, un même élément apparaissant sur des figures différentes présente une référence unique.

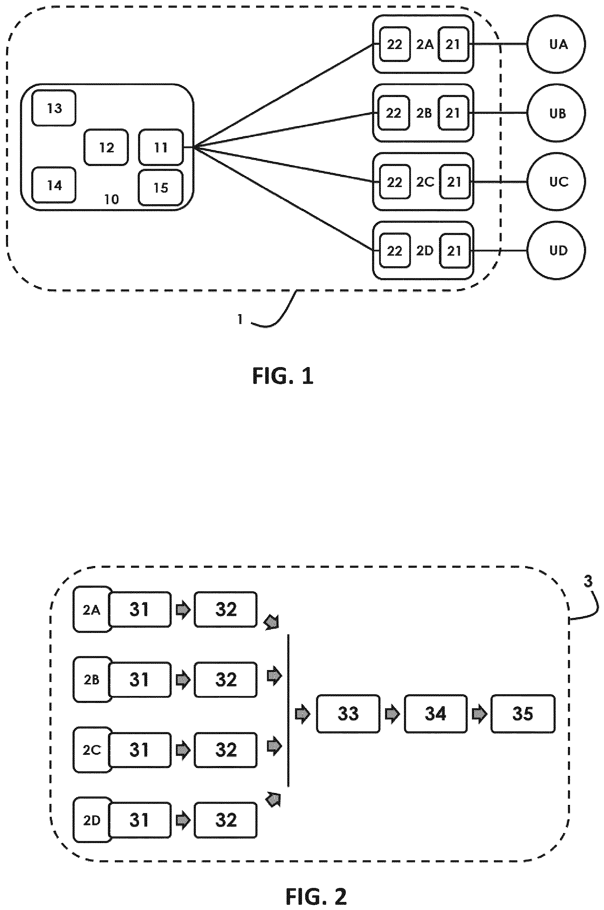

[Fig. 1] montre une représentation schématique d'un système centralisé d'analyse de la déglutition d'une pluralité d'utilisateurs selon l'invention.

Le système centralisé 1 d'analyse de la déglutition d'une pluralité d'utilisateurs représenté à la [Fig. 1] comprend un dispositif central de gestion 10 et une pluralité de dispositifs de détection de la déglutition 2A, 2B, 2C et 2D. Pour une simplicité d'utilisation du système 1 et pour ne pas avoir à traiter de trop grandes quantités de données, le système 1 comprend préférentiellement entre 5 et 10 dispositifs de détection de la déglutition. Cependant, et bien que la [Fig. 1] représente quatre dispositifs de détection de la déglutition, la présente invention couvre tout système comprenant au moins deux dispositifs de détection de la déglutition.

Chaque dispositif de détection de la déglutition 2A, 2B, 2C et 2D est associé à au moins un utilisateur, préférentiellement un unique utilisateur. Par exemple, comme représenté à la [Fig. 1], le dispositif de détection de la déglutition 2A est associé à l'utilisateur UA, le dispositif de détection de la déglutition 2B est associé à l'utilisateur UB, le dispositif de détection de la déglutition 2C est associé à l'utilisateur UC et le dispositif de détection de la déglutition 2D est associé à l'utilisateur UD. On entend par « associé » à un utilisateur le fait que le dispositif de détection de la déglutition 2A à 2D permet de mesurer un signal de déglutition de l'utilisateur associé UA à UD. Un dispositif de détection de la déglutition 2A à 2D peut être porté par l'utilisateur associé UA à UD, par exemple dans le mode de réalisation préférentiel dans lequel le dispositif de détection de la déglutition 2A à 2D est un dispositif collier.

Chaque dispositif de détection de la déglutition 2A à 2D comprend un capteur de détection de la déglutition 21 et une interface d'envoi de données 22. Le capteur de détection de la déglutition 21 est configuré pour mesurer un signal de déglutition de l'utilisateur auquel il est relié. Ce capteur de détection de déglutition 21 peut par exemple être un accéléromètre situé au niveau du larynx. Il peut alors mesurer un signal de déglutition de l'utilisateur UA, par exemple un signal de mouvement laryngé correspondant à une déglutition ou tout autre mouvement permettant d'analyser une déglutition. Le capteur de détection de déglutition 21 peut par exemple être un microphone, le signal de déglutition de l'utilisateur mesuré étant alors un son laryngé, ou tout autre son permettant d'analyser une déglutition. Le capteur de détection de déglutition 21 peut être tout capteur capable de mesurer un signal de déglutition permettant d'analyser une déglutition de l'utilisateur. En outre, le dispositif de détection de la déglutition 2A à 2D peut comprendre une pluralité de capteurs de détection de déglutition 21, par exemple une combinaison d'un microphone et d'un accéléromètre pour améliorer la précision et la fiabilité de la détection de la déglutition. Les dispositifs de détection de la déglutition 2A à 2D ne sont pas forcément identiques en ce qu'ils peuvent comprendre des capteurs de détection de la déglutition 21 différents d'un dispositif de détection de la déglutition 2A à 2D à un autre. Ainsi, la présente invention permet de s'adapter à des dispositifs de détection de la déglutition existants, et aux dispositifs de détection de la déglutition 2A à 2D d'être adaptés aux utilisateurs, en comprenant certains capteurs de détection de la déglutition 21 particuliers pour certains utilisateurs et d'autres capteurs de détection de la déglutition 21 pour d'autres utilisateurs.

Chaque dispositif de détection de la déglutition comprend une interface d'envoi de données 22, configurée pour envoyer les signaux de déglutition mesurés par le capteur de détection de la déglutition 21 à travers un réseau ou directement via une connexion filaire ou sans fil au dispositif central de gestion 10 du système centralisé 1. Cette transmission eut être réalisée en Bluetooth^{®}, en Wi-Fi^{®}, ou selon tout autre protocole de transfert de données sans-fil ou filaire, préférentiellement sans-fil, afin de pouvoir ajouter et supprimer des dispositifs de détection de la déglutition 2A à 2D au système centralisé 1 de manière simple et afin de laisser leur liberté de mouvement aux utilisateurs UA à UD. L'interface d'envoi de données 22 peut en outre permettre la réception de données en étant configurée pour recevoir des données. Cela peut permettre de réaliser des mises à jour à distance des dispositifs de détection de la déglutition, et/ou d'envoyer des commandes aux dispositifs de détection de la déglutition 2A à 2D. De telles commandes peuvent par exemple être une commande de mesure, une commande liée à la gestion de la batterie, ou tout autre type de commande.

En outre, les dispositifs de détection de la déglutition 2A à 2D peuvent chacun comprendre au moins un capteur parmi un capteur de fréquence cardiaque, un capteur de température du corps, un capteur de sudation, un capteur de son de respiration, un capteur de fréquence respiratoire, un capteur d'activité musculaire (non représentés). Les dispositifs de détection de la déglutition 2A à 2D représentés à la [Fig. 1] peuvent comprendre tous les capteurs précédemment cités, mais ils peuvent aussi ne comprendre qu'un des capteurs précédemment cités ou toute combinaison possible des capteurs précédemment cités. Les capteurs précédemment cités permettent de connaître précisément l'état des utilisateurs UA à UD pendant un exercice de rééducation à la déglutition ou pendant un examen de la déglutition d'un ou des utilisateurs. Ils permettent en outre de réaliser une meilleure analyse de la déglutition de l'utilisateur lorsque la déglutition de plusieurs utilisateurs est analysée simultanément, comme il sera expliqué plus loin dans la description.

Le système centralisé 1 d'analyse de la déglutition d'une pluralité d'utilisateurs UA à UD comprend un dispositif central de gestion 10 connecté aux dispositifs de détection de la déglutition 2A à 2D.

Le dispositif central de gestion 10 selon l'invention, et comme représenté à la [Fig. 1], comprend un module de réception 11, un processeur 12, un module de stockage 13 et un module d'affichage 14. Le dispositif central de gestion 10 peut comprendre en outre un module d'alarme 15.

Le module de réception 11 du dispositif central de gestion 10 est configuré pour recevoir, de chaque dispositif de détection de la déglutition 2A à 2D, les signaux de déglutition mesurés par leurs capteurs 21 respectifs. Pour cela, le module de réception 11 utilise le même protocole que l'interface d'envoi de données 22 de chaque dispositif de détection de la déglutition 2A à 2D. Les dispositifs de détection de la déglutition peuvent comprendre des interfaces d'envoi de données 22 différentes ou utilisant des protocoles différents. Dans un tel cas, le module de réception 11 du dispositif central de gestion 10 est capable de gérer les différents protocoles ou de recevoir des données de chacune des interfaces d'envoi de données 22, par exemple en comprenant des sous-modules adaptés à chacun des protocoles ou à chacune des interfaces d'envoi de données 22. Ainsi, le dispositif central de gestion peut s'adapter à des dispositifs de détection de la déglutition 2A à 2D différents et donc s'adapter à des dispositifs 2A à 2D déjà existants et utilisés. Cela permet aux utilisateurs habitués à un dispositif de détection de la déglutition de ne pas avoir à en changer pour implémenter le système centralisé 1.

Le dispositif central de gestion 10 comprend au moins un processeur 12. Le processeur 12 est configuré pour réaliser une analyse de chacun des signaux de déglutition reçus. On entend par « analyse » le fait d'étudier un signal de déglutition. On entend par « résultat d'analyse » le fait de donner un attribut à un signal de déglutition. Un exemple d'analyse est la caractérisation d'un signal de déglutition. Un autre exemple d'analyse, qui est aussi un mode de réalisation préférentiel, est la classification du signal de déglutition, par exemple dans une classe correspondant à une déglutition normale ou dans une classe correspondant à une déglutition anormale. Cette classification peut être réalisée de manière connue de l'homme du métier par un algorithme d'apprentissage automatique utilisant un modèle statistique ou encore un réseau de neurones. Les signaux issus des capteurs 21 peuvent permettre au modèle de classification d'être plus précis et plus fiable dans ses prises de décision pour classifier un signal de déglutition comme étant « anormal », c'est-à-dire représentatif d'une dysphagie de l'utilisateur étudié, par exemple une déglutition présentant un risque de fausse-route ou d'aspiration, ou « normal », c'est-à-dire représentatif d'une déglutition non caractéristique d'une dysphagie de l'utilisateur étudié. Le dispositif central de gestion 10 peut comprendre une pluralité de processeurs 12, par exemple pour être en mesure de traiter plusieurs signaux de déglutition reçus simultanément. Le dispositif centralisé de gestion 10 peut analyser chaque signal en utilisant un modèle unique apprenant grâce aux données de l'ensemble des dispositifs de détection de la déglutition « satellites » 2A à 2D, ou une pluralité de modèles, chaque modèle étant dédié à un ou plusieurs utilisateurs. Cela peut permettre une meilleure adaptabilité à des profils d'utilisateurs différents et une meilleure analyse de la déglutition. Dans une variante, le dispositif central de gestion 10 peut analyser chaque signal en utilisant un modèle unique apprenant à partir des données de l'ensemble des dispositifs de détection de la déglutition « satellites » 2A à 2D et à partir de données de l'ensemble des dispositifs centraux de gestion 10 lorsque ceux-ci sont reliés par un réseau. Ainsi, lorsque des dispositifs centraux de gestion 10 sont utilisés par plusieurs établissements différents ou dans des localités différentes, ceux-ci peuvent être reliés par un réseau et apprendre à partir des données de l'ensemble des utilisateurs de tous les dispositifs de détection de la déglutition « satellites » connectés à tous les dispositifs centraux de gestion 10. Cela permet un apprentissage plus rapide car la quantité de données d'apprentissage est plus importante.

L'analyse des signaux de déglutition peut être utilisée par exemple pour adapter en temps réel ou au fur et à mesure des analyses les repas pris par les utilisateurs UA à UD. Ainsi, dans le cadre d'analyses de signaux de déglutition dans une classe « anormale », il est possible d'adapter la texture ou la quantité des aliments proposés à l'utilisateur UA à UD pour améliorer la déglutition de l'utilisateur UA à UD, par exemple en proposant des aliments plus « mous », ou en proposant des quantités moins importantes d'aliments. Des tests peuvent être réalisés pour atteindre une alimentation optimale pour la déglutition des utilisateurs UA à UD.

Une fois analysés, les signaux reçus des dispositifs de détection de déglutition 2A à 2D sont stockés par un module de stockage 133 du dispositif central de gestion 10. Le signal analysé est stocké conjointement au résultat de son analyse, c'est-à-dire à son ou ses attributs attribués par le processeur 12. Les signaux peuvent être stockés associés à un identifiant utilisateur, c'est-à-dire un identifiant de l'utilisateur pour lequel le signal de déglutition a été mesuré. Cela permet de pouvoir accéder à l'ensemble des signaux de déglutition mesurés et reçus pour un utilisateur particulier, et ainsi d'analyser l'évolution temporelle de la déglutition d'un utilisateur parmi la pluralité d'utilisateurs UA à UD. Cette évolution temporelle permet de réaliser des bilans de déglutition pour les différents utilisateurs UA à UD, et de prendre en compte l'évolution de la déglutition des utilisateurs UA à UD par exemple pour adapter l'alimentation des utilisateurs UA à UD.

Le module de stockage 13 du dispositif central de gestion 10 peut être un module « temporaire », c'est-à-dire un module de stockage pendant une durée donnée, par exemple une semaine, avant transmission des données stockées à une base de données avec plus de capacité de stockage. Cette transmission peut être réalisée à travers un réseau de communication.

Le dispositif central de gestion 10 peut aussi comprendre une interface réseau (non représentée). Une telle interface réseau peut être utilisée pour envoyer et recevoir à travers un réseau des signaux de déglutition afin de permettre à d'autres dispositifs centraux de gestion 10 reliés au réseau d'apprendre à partir de ces signaux de déglutition. Les dispositifs centraux de gestion 10 peuvent aussi utiliser un même modèle unique localisé au niveau d'un dispositif accessible via le réseau, le modèle apprenant à partir des données reçues de chacun des dispositifs centraux de gestion 10 reliés au réseau. Une interface réseau du dispositif central de gestion 10 peut aussi être utilisée pour envoyer à travers un réseau les résultats d'analyse des signaux de déglutition réalisée par le dispositif central de gestion 10 afin de permettre à un praticien d'analyser ces résultats à distance, par exemple dans le cadre d'un bilan de déglutition.

Dans un autre mode de réalisation, le module de stockage 13 du dispositif central de gestion 10 n'est pas physiquement présent dans ou proche du dispositif central de gestion 10 mais est accessible à travers un réseau, permettant une sécurité supplémentaire concernant la persistance des données et un accès aux données à distance. Pour cela, le dispositif central de gestion 10 peut comprendre une interface réseau pour envoyer et recevoir des données à travers un réseau.

En outre, le dispositif central de gestion 10 comprend un module d'affichage 14, configuré pour afficher au moins un signal de déglutition stocké et/ou l'analyse du au moins un signal de déglutition stocké. Le module d'affichage 14 peut être un écran ou tout autre type de dispositif permettant d'afficher des données à un utilisateur du dispositif central de gestion 10 tel qu'un praticien.

Préférentiellement, les signaux de déglutition et leur résultat d'analyse attribué par le processeur 12 sont affichés conjointement par le module d'affichage 14. Préférentiellement encore, les signaux de déglutition reçus peuvent être affichés en temps réel ou en quasi-temps-réel, c'est-à-dire par exemple dès leur réception par le module de réception 11, après analyse de ces signaux par le processeur 12, ou après stockage par le module de stockage 13. Les signaux de déglutition reçus peuvent être affichés sans leur résultat d'analyse, par exemple avant que l'analyse ait été réalisée par le processeur 12, et l'analyse peut être affichée par la suite par le module d'affichage 14, lorsqu'elle est disponible, c'est-à-dire lorsqu'elle a été réalisée par le processeur 12 ou lorsqu'elle a été stockée par le module de stockage 13.

Préférentiellement, les signaux reçus de plusieurs dispositifs de détection de la déglutition 2A à 2D sont affichés simultanément par le module d'affichage 14. Cela permet d'avoir une vue d'ensemble de la déglutition de la pluralité d'utilisateurs UA à UD. Comme expliqué au paragraphe précédent, chaque signal de déglutition associé à un utilisateur peut être affiché conjointement avec son résultat d'analyse, et ce pour plusieurs signaux reçus de plusieurs dispositifs de détection de la déglutition 2A à 2D ou reçus du même dispositif de détection de la déglutition. Ainsi, il est possible d'analyser la déglutition d'une pluralité d'utilisateur UA à UD simultanément et même de les comparer en temps réel ou en quasi-temps-réel, sans avoir à analyser chaque utilisateur un par un ni à se déplacer auprès de chacun des utilisateurs un par un.

Dans un mode de réalisation préférentiel, le dispositif central de gestion 10 comprend en outre un module d'alarme 15.

Le module d'alarme 15 est configuré pour émettre une alarme lorsque le résultat d'analyse d'un des signaux de déglutition reçus vérifie une règle prédéfinie.

Une règle prédéfinie pour l'émission d'une alerte peut par exemple être vérifiée, dans le mode de réalisation dans lequel l'analyse du signal de déglutition est une classification, lorsque le signal de déglutition est classé dans une ou plusieurs classes prédéfinies. Par exemple, la règle prédéfinie peut être vérifiée lorsque le signal est classé dans une classe correspondant à une déglutition « anormale ». D'autres exemples de règles prédéfinies peuvent comprendre une perte de connexion entre le dispositif central de gestion 10 et un dispositif de détection de la déglutition 2A à 2D, le fait que le signal de déglutition se soit vu attribuer un certain attribut, ou toute autre règle liée à une événement nécessitant l'attention d'un utilisateur du dispositif central de gestion 10.

Cette alarme émise par le module d'alarme 15 lorsqu'une règle prédéfinie est vérifiée peut être une alerte affichée sur le module d'affichage 14, par exemple sous la forme d'une mise en couleur ou en surbrillance du ou des attributs compris dans le résultat d'analyse du signal. Lorsque l'attribut est une classe dans laquelle le signal de déglutition a été classé, l'affichage par le module d'affichage 14 d'une classe correspondant à une déglutition « anormale » peut être réalisé en rouge, et l'affichage par le module d'affichage 14 d'une classe correspondant à une déglutition « normale » peut être réalisé en vert. En outre, l'affichage d'une classe correspondant à une déglutition « anormale » peut comprendre une animation telle qu'un clignotement de texte ou toute autre animation permettant d'attirer l'attention d'un utilisateur du dispositif central de gestion 10 sur le signal analysé. Une alarme peut aussi être une alerte sonore via un système ou un dispositif de sonorisation tel qu'au moins un haut-parleur. Préférentiellement, une alerte permet d'attirer rapidement l'attention d'un utilisateur du dispositif central de gestion 10 sur des informations importantes en lien avec l'événement nécessitant l'attention de l'utilisateur. Par exemple, lorsque l'évènement signalé par l'alarme est une classification particulière d'un signal de déglutition d'un utilisateur, les informations importantes peuvent être une information d'identification de l'utilisateur, la classe dans laquelle le signal a été classé, et/ou l'heure à laquelle l'événement a eu lieu. L'alarme peut aussi être un signal visuel tel qu'un signal lumineux, ou toute autre alarme attirant l'attention de l'utilisateur du dispositif central de gestion 10. Un tel utilisateur du dispositif central de gestion 10 peut par exemple être un praticien ou un infirmier.

Bien que non représenté, les dispositifs de détection de la déglutition 2A à 2D peuvent être associés à un dispositif mobile du patient, tel qu'un smartphone ou un ordinateur portable. Le module de réception 11 du dispositif central de gestion 10 peut alors être configuré en outre pour envoyer des données aux dispositifs mobiles des utilisateurs UA à UD, soit directement en étant connecté aux dits dispositifs mobiles, soit via le dispositif de détection de la déglutition de l'utilisateur. Ainsi, chaque utilisateur UA à UD peut utiliser son dispositif de détection de la déglutition 2A à 2D de manière classique, sans avoir connaissance de la présence du dispositif central de gestion 10.

La [Fig. 2] montre une représentation schématique d'un procédé d'analyse de la déglutition d'une pluralité d'utilisateurs selon l'invention.

Le procédé 3 d'analyse de la déglutition d'une pluralité d'utilisateurs UA à UD comprend une première étape de mesure d'un signal de déglutition 31. Cette étape 31 comprend la mesure, par un capteur de détection de la déglutition 21 de chaque dispositif de détection de la déglutition de la pluralité de dispositifs de détection de la déglutition UA à UD.

Pour chaque dispositif de détection de la déglutition 2A à 2D, le procédé 3 d'analyse de la déglutition d'une pluralité d'utilisateurs UA à UD comprend une deuxième étape d'envoi du signal de déglutition mesuré par le ou les capteurs de détection de la déglutition 21 au dispositif central de gestion 10, par l'interface d'envoi de données 22. Préférentiellement, cet envoi est réalisé immédiatement après mesure du signal de déglutition. Il est aussi possible, dans une variante non préférée de l'invention, qu'un, plusieurs, ou chaque dispositif de détection de la déglutition 2A à 2D comprenne un module de stockage permettant de stocker les signaux mesurés et les envoie par groupe de plusieurs signaux de déglutition.

Le procédé 3 d'analyse de la déglutition d'une pluralité d'utilisateurs UA à UD comprend une troisième étape 33 de réception, par le module de réception 11 du dispositif central de gestion 10, des signaux de déglutition envoyés par les dispositifs de détection de la déglutition 2A à 2D. Comme indiqué précédemment, les étapes 32 d'envoi et 33 de réception sont réalisées selon le protocole réseau filaire ou sans-fil utilisé, par exemple Bluetooth^{®}, Wifi^{®} etc.

Le procédé 3 d'analyse de la déglutition d'une pluralité d'utilisateurs UA à UD comprend une quatrième étape 34 d'analyse de chaque signal de déglutition reçu par le processeur 12 du dispositif central de gestion 10. Comme indiqué précédemment, l'analyse comprend l'attribution d'un attribut au signal, par exemple un label, une valeur sur une échelle, ou, préférentiellement une classe résultant d'une classification.

Dans une cinquième étape 35 du procédé 3 d'analyse de la déglutition d'une pluralité d'utilisateurs, une alarme est émise par le module d'alarme 15 du dispositif central de gestion 10, lorsqu'au moins un signal de déglutition parmi les signaux de déglutition reçus vérifie une règle prédéfinie, et préférentiellement, lorsque la règle est vérifiée quand il est classifié dans au moins une classe prédéfinie. Comme indiqué précédemment, l'alarme peut être visuelle et/ou sonore ou de tout autre type permettant d'attirer l'attention d'un utilisateur du dispositif central de gestion 10.

## Revendications

1. Système centralisé d'analyse de la déglutition d'une pluralité d'utilisateurs, le système étant **caractérisé en ce qu'**il comprend :
- Une pluralité de dispositifs de détection de la déglutition, chaque dispositif de détection de la déglutition comprenant au moins un capteur de détection de déglutition configuré pour mesurer un signal de déglutition d'un utilisateur de la pluralité d'utilisateurs,
- Au moins un dispositif central de gestion connecté aux dispositifs de détection de la pluralité de dispositifs de détection et comprenant au moins :
∘ un module de réception configuré pour recevoir les signaux de déglutition de la pluralité de dispositifs de détection de la déglutition,
∘ au moins un processeur configuré pour réaliser une analyse de chacun des signaux de déglutition reçus,
∘ un module de stockage configuré pour stocker chacun des signaux de déglutition reçus et le résultat de l'analyse de chacun des signaux de déglutition reçus réalisée par le processeur,
∘ un module d'affichage configuré pour afficher au moins un signal stocké et/ou le résultat de l'analyse du au moins un signal stocké.

2. Système centralisé selon la revendication précédente **caractérisé en ce que** le dispositif central de gestion comprend en outre un module d'alarme configuré pour émettre une alarme lorsque le résultat de l'analyse d'un des signaux de déglutition reçus vérifie une règle prédéfinie.

3. Système centralisé selon la revendication précédente **caractérisé en ce que** le module d'alarme est configuré pour vérifier la règle prédéfinie, la règle prédéfinie étant vérifiée lorsque le signal de déglutition reçu est classifié dans au moins une classe prédéfinie.

4. Système centralisé selon l'une quelconque des revendications précédentes **caractérisé en ce que** le module de stockage est configuré pour stocker chacun des signaux de déglutition reçus et le résultats de l'analyse de chacun des signaux de déglutition reçus en association avec un identifiant utilisateur.

5. Système centralisé selon l'une quelconque des revendications précédentes **caractérisé en ce que** le capteur de détection de déglutition est un microphone pour détecter un son de déglutition et/ou un accéléromètre pour détecter un mouvement de déglutition.

6. Système centralisé selon l'une quelconque des revendications précédentes **caractérisé en ce que** les dispositifs de détection de la déglutition comprennent chacun en outre au moins un capteur parmi les capteurs de fréquence cardiaque, de température du corps, de sudation, de son de respiration, de fréquence respiratoire, d'activité musculaire.

7. Procédé d'analyse de la déglutition d'une pluralité d'utilisateurs, chaque utilisateur utilisant un dispositif de détection de la déglutition, le procédé comprenant les étapes de :
- Mesure d'au moins un signal de déglutition par chaque dispositif de détection de déglutition ;
- Envoi des signaux de déglutition par chaque dispositif de détection de déglutition à un module de réception d'un dispositif central de gestion ;
- Analyse de chaque signal de déglutition reçu par un processeur du dispositif central de gestion.

8. Procédé d'analyse de la déglutition d'une pluralité d'utilisateurs selon la revendication précédente **caractérisé en ce que** l'étape d'analyse de chaque signal de déglutition reçu comprend la classification de chaque signal de déglutition reçu.

9. Procédé d'analyse de la déglutition d'une pluralité d'utilisateurs selon la revendication précédente **caractérisé en ce qu'**il comprend en outre une étape d'émission d'une alarme, par un module d'alarme du dispositif central de gestion, lorsqu'au moins un signal de déglutition parmi les signaux de déglutition reçus est classifié dans au moins une classe prédéfinie.
